# EUROPÄISCHE PATENTSCHRIFT

(11) **EP 0 630 617 B1**
(45) Veröffentlichungstag und Bekanntmachung des Hinweises auf die Patenterteilung: **02.09.1998**
(21) Anmeldenummer: 93110061.4
(22) Anmeldetag: 24.06.1993
(51) Int. Cl.: A61B 17/22

(54) **Aspirationskatheteranordnung**
Suction catheter assembly
Dispositif formant sonde d'aspiration

(43) Veröffentlichungstag der Anmeldung: 28.12.1994
(73) Patentinhaber: Schneider (Europe) GmbH, 8180 Bülach (CH)
(72) Erfinder: Cramer, M.B. P.D. Dr. med, Heusenerstrasse 40 D-42283 Wuppertal (DE)
(74) Vertreter: Rottmann, Maximilian R.

(56) Entgegenhaltungen:
- EP-A- 0 025 704
- EP-A- 0 245 211
- WO-A-90/04994
- FR-A- 2 517 973
- US-A- 5 011 488

## Beschreibung

Die Erfindung betrifft eine Aspirationskatheteranordnung zum Entfernen von Thromben und Emboli aus Blutgefässen.

Gattungsgemässe Katheteranordnungen werden zum Entfernen von Thromben und Emboli eingesetzt, indem der Katheter über ein Blutgefäss an das zu entfernende Blutgerinnsel herangeführt und mit seinem proximalen Ende an eine Absaugvorrichtung angeschlossen wird. Durch den im Katheter herrschenden Unterdruck wird dann das Blutgerinnsel an- und abgesaugt. Ein grosses Problem beim Absaugen von Blutgerinnseln mit solchen Aspirationskathetern besteht darin, dass der Aspirationskatheter durch die Blutgerinnsel, insbesondere wenn diese einen grösseren Durchmesser als der Katheter selber haben, leicht verstopft. Um eine solche Verstopfung zu entfernen, muss der Katheter aus dem Blutgefäss entfernt werden. Dieser verstopfte Katheter wird dann entweder ausserhalb des Körpers gereinigt oder es wird ein neuer ungebrauchter Katheter in das Gefäss eingeführt. In beiden Fällen muss ein Katheter jedoch mindestens zweimal in das entsprechende Blutgefäss eingeführt werden.

Jedes Vorschieben des Katheters im Blutgefäss stellt jedoch eine Belastung für die Gefässwand dar. Auch wenn zur Verringerung der Verletzungsgefahr der Gefässwand der Katheter mit eingeschobenem Dilator vorgeschoben wird, das Vorschieben muss auf jeden Fall langsam erfolgen. Der an sich schon aufwendige Reinigungsvorgang wird dadurch zusätzlich verlängert. Ausserdem muss beim neuen Einführen des Katheters die Lage des Blutgerinnsels innerhalb des Gefässsystems immer wieder neu gefunden werden. Falls ein bis dahin ungebrauchter Katheter neu eingesetzt wird, muss dieser ebenso wie der erste eingesetzte Katheter vollkommen steril sein.

Um die Verstopfungsgefahr solcher Aspirationskatheter beim Absaugen von Blutgerinnseln zu vermindern, sind verschiedene Ausführungsformen von Kathetern bekannt. In der EP 0 177 782 ist beispielsweise ein Apparat zum transluminalen Entfernen von Thromben beschrieben, welcher eine rotierende Welle aufweist. Durch diese rotierende Welle soll das Fibrin aus dem Thrombus aufgenommen werden und der Thrombus soll dadurch aufgebrochen werden, so dass das Blut wieder fliessen kann. Eine besonders abgerundete Spitze an der Welle selbst und der Katheter aussen um die Welle herum verhindern eine Verletzung der Gefässwand durch die Welle. Über den Katheter kann der Druck am Behandlungsort gemessen werden und es können Medikamente eingegeben werden. Vor allem kann über den Katheter auch kontinuierlich Flüssigkeit abgesaugt werden. Das an der Welle aufgewickelte Fibrin muss allerdings mechanisch von der Welle entfernt werden, dazu muss die Welle aus dem Katheter herausgezogen werden.

Im weiteren ist aus der EP 0 367 982 eine als Vorrichtung zur perkutanen Entfernung von Thromben und Emboli bezeichnete Katheteranordnung bekannt, welche im distalen Ende eines Katheters über einen rotierenden Propeller zum Zerkleinern des Blutgerinnsels beim Einsaugen verfügt. Über den Katheter können mit Hilfe einer Absaugvorrichtung die Blutgerinnsel kontinuierlich abgesaugt werden. Beiden Ausführungsformen haften jedoch einige Nachteile an. So wird durch den Rotationskörper, wie auch durch die zu dessen Antrieb notwendige Antriebswelle, der für die Absaugung zur Verfügung stehende Katheterquerschnitt wesentlich reduziert. Im weiteren sind solchermassen ausgebildete Katheter, durch den Rotationskörper und die zu dessen Antrieb notwendige Antriebswelle, relativ kompliziert aufgebaut und aufwendig in der Herstellung. Vor allem aber weisen diese Instrumente keinen Dilator auf, sie müssen deshalb mit einem besonders für diese Zwecke bereit zu haltenden Einführbesteck in das Blutgefäss eingeführt werden. Ebenfalls wegen der Abwesenheit eines Dilators besteht bei diesen Instrumenten beim Vorschieben durch das Gefäss eine Verletzungsgefahr für die Gefässwand, hervorgerufen durch den Rand der distalen Katheteröffnung. Ausserdem kann durch einen rotierenden Zerkleinerungskopf bzw. eine Spitze, eine Verletzung von umliegenden Gefässteilen nicht ganz ausgeschlossen werden. Bei der Benutzung eines Einführungsbestecks wird das Blutgefäss zunächst mit einer hohlen Punktionsnadel punktiert. In der hohlen Punktionsnadel wird dann ein kurzer relativ stabiler Führungsdraht in das Blutgefäss vorgeschoben. Über diesen kurzen Führungsdraht wird ein kegelförmiger Dilator geschoben, der den Führungsdraht in sich in einer zentralen Längsbohrung aufnimmt. Der kegelförmige Dilator weitet die Punktionsstelle soweit auf, dass über den Dilator eng anliegend der Einführungskatheter geschoben werden kann. Der Dilator und der Einführungskatheter sind genau aufeinander abgestimmt und müssen mit ihrem Durchmesser so ausgewählt werden, dass durch den Einführungskatheter dann der entsprechende Aspirationskatheter in das Blutgefäss eingeschoben werden kann.

Für einen einmaligen Einsatz sind solche Ausführungsformen von Kathetern ausserdem sehr teuer. Sollen sie jedoch mehrmals eingesetzt werden, so muss die gesamte Katheteranordnung aufwendig gereinigt und neu sterilisiert werden. Da eine solche Anordnung einem gewissen Verschleiss unterliegt und die mehrfache Sterilisierung zu Veränderungen des Materials führen kann, ist ein mehrmaliger Einsatz jedoch mit gewissen Risiken verbunden. Zudem sind Katheteranordnungen mit rotierenden Körpern auch noch relativ störanfällig. Auch kann eine solcherart ausgebildete Katheteranordnung mit ihrem durch die Antriebswelle steifen, distalen Ende nicht um enge Kurvenradien herumgeführt werden. Schliesslich weisen solche Vorrichtungen, prinzipbedingt, einen relativ grossen Aussendurchmesser auf, so dass nur entsprechend grosslumige Gefässe dafür zugänglich sind.

Aus dem DE GBM 8910603.2 ist eine Vorrichtung zum Ausbringen von Blutgerinnseln aus Arterien und Venen bekannt. Diese Vorrichtung weist einen Innenkatheter auf, welcher an seinem distalen Ende mit einem aufblähbaren Ballon versehen ist (Fogarty-Katheter). Dieser Innenkatheter wird von einem Schleusenkatheter umfasst, der ein radial aufweitbares Endstück besitzt. Der Schleusenkatheter wiederum, wird von einem Aussenkatheter umfasst. Zum Ausbringen von Blutgerinnseln wird die Vorrichtung in der bekannten Seldinger-Technik, unter Zuhilfenahme eines Einführbestecks, perkutan in das Blutgefäss eingeführt. Anschliessend wird der am Ende des Innenkatheters angeordnete Ballon in nicht aufgeblähtem Zustand durch das Blutgerinnsel geschoben und danach aufgebläht. Der Thrombus wird sodann mechanisch durch Zug am Innenkatheter mit dem aufgeblähten Ballon in das aufgeweitete Endstück des Schleusenkatheters gezogen, durch Zusammenfalten des radial aufweitbaren Endstücks ausgepresst und durch mechanisches Herausziehen des Schleusenkatheters mit dem Innenkatheter aus dem Blutgefäss entfernt.

Durch den am Ende radial aufweitbaren Schleusenkatheter hat diese Vorrichtung den Vorteil, dass mit ihr Blutgerinnsel, die grösser im Durchmesser sind als der Schleusenkatheter, in diesen hineingezogen und aus dem Gefäss entfernt werden können. Diese Vorrichtung ist jedoch auf solche Anwendungsfälle eingeschränkt, in denen Blutgerinnsel stückweise aus dem Gefäss entfernt werden sollen. Obwohl an den Schleusenkatheter unterstützend ein Unterdruck angelegt werden kann, ist diese Vorrichtung für einen kontinuierlichen Abtransport der Blutgerinnsel durch Absaugung trotzdem nicht geeignet. Das Fibrin des Thrombus bleibt beim Ansaugen in dem aufweitbaren Geflecht am Ende des Schleusenkatheters hängen und kann durch den Unterdruck dort nur festgehalten werden, es kann nicht kontinuierlich weiter abgesaugt werden.

Der Schleusenkatheter weist zudem im Bereich seines distalen Endes nur ein sehr kleines Lumen auf, da er in diesem Bereich das Geflecht des aufweitbaren Endstücks tragen muss und die Wandstärke zum Übertragen der zum Bewegen dieses Geflechts notwendigen Schub- und Zugkräfte entsprechend gross dimensioniert sein muss.

Ein weiterer Nachteil einer solchen Vorrichtung ist darin zu sehen, dass der Schleusenkatheter und der Aussenkatheter zumindest im Bereich ihrer distalen Enden durch das aufweitbare Geflecht relativ steif ausgebildet sind. Mit dieser Vorrichtung können daher nur Gefässwindungen mit entsprechend grossen Krümmungsradien überwunden werden. Verschlungene Gefässverläufe mit kleinen Kurvenradien können jedoch mit einer solcherart ausgebildeten Vorrichtung nicht durchkreuzt bzw. erreicht werden. Durch eine unsachgemässe Aufweitung des am distalen Ende des Schleusenkatheters angebrachten Geflechts besteht zudem die Gefahr, dass dadurch die Blutgefässe verletzt werden.

Es ist somit die Aufgabe der Erfindung, eine ohne separates Einführbesteck einsetzbare Katheteranordnung zum Entfernen von Thromben und Emboli aus Blutgefässen durch kontinuierliche Absaugung zu schaffen, welche einfach in der Anwendung und unkompliziert im Aufbau ist, welche kostengünstig hergestellt werden kann und welche auch bei Verstopfungen des Absaugkatheters noch eine hohe Arbeitsgeschwindigkeit zulässt und die ausserdem im Bezug auf die Beanspruchung der Blutgefässe, in die sie eingeführt wird, schonend eingesetzt werden kann, und bei welcher auch Blutgerinnsel, die grösser sind als der Durchmesser des Absaugkatheters, noch vollständig entfernt werden können, die ferner im Verhältnis zu ihren Aussendurchmesser einen grossen Absaugquerschnitt bietet, die flexibel auch durch enge Kurven in der Gefässbahn geführt werden kann und die auch noch in kleine Gefässe vorgeschoben werden kann, die aber am proximalen Ende zum Vorschieben trotzdem über eine hohe Steifigkeit verfügt und die eine glatte Ansaugöffnung aufweist, ohne Hindernisse für den Thrombus.

Diese Aufgabe wird durch eine Aspirationskatheteranordnung gelöst, welche die im Patentanspruch 1 aufgeführten Merkmale besitzt. Weitere vorteilhafte Ausführungsformen sind in den abhängigen Ansprüchen 2 bis 10 umschrieben.

Die erfindungsgemässe Aspirationskatheteranordnung weist einen Zentralkatheter (2), der zur Aufnahme eines axial verschiebbaren Dilators (3) ausgebildet ist sowie einem den Zentralkatheter (2) koaxial umfassenden Aussenkatheter (1) auf, wobei die Aspirationskatheteranordnung zum kontinuierlichen Absaugen von Thromben und Emboli ausgebildet ist, indem der Zentralkatheter (2) während der kontinuierlichen Absaugung einen bis zu seinem distalen Ende gleichbleibenden Innendurchmesser aufweist, wobei das distale Ende des Aussenkatheters (1) bis an das distale Ende des Zentralkatheters (2) heranschiebbar ist, und wobei der Aussendurchmesser des Aussenkatheters (1) nur unwesentlich grösser als der des Zentralkatheters (2) ist, so dass der zum Absaugen zur Verfügung stehende Querschnitt annähernd dem Querschnitt des Aussenkatheters (1) entspricht.

Durch eine solche Ausgestaltung der Aspirationskatheteranordnung kann diese perkutan ohne Einführbesteck, durch eine kleine Punktionsöffnung, in ein entsprechendes Blutgefäss eingeführt werden. Normalerweise fluchten die distalen Enden der beiden Katheter während des Einführens. Es ist aber auch denkbar, dass der eine der beiden Katheter den anderen an dessen distalem Ende überragt. Dadurch kann die Steifigkeit der Katheteranordnung und ihr Durchmesser am distalen Ende sehr einfach variiert werden, ohne dass dadurch die gewünschte hohe Steifigkeit im Bereich des proximalen Endes der Katheteranordnung oder der grosse Absaugquerschnitt beeinträchtigt würden. Zum Entfernen eines Blutgerinnsels wird der Aussenkatheter, zusammen mit dem Zentralkatheter und mit eingesetztem Dilator und Führungsdraht, in bekannter Weise durch die Punktionsstelle in das Blutgefäss eingeführt und in diesem weiter an das zu entfernende Blutgerinnsel herangeschoben. Anschliessend wird der Dilator und der Führungsdraht entfernt.

Durch Anschliessen des Zentralkatheters an eine Absaugvorrichtung wird nun versucht, das Blutgerinnsel abzusaugen. Solange der Zentralkatheter durch das abzusaugende Blutgerinnsel nicht verstopft, unterscheidet sich der Absaugvorgang nicht von den herkömmlichen Absaug-Methoden. Verstopft jedoch das Blutgerinnsel den Zentralkatheter, was bei grösseren Thromben sehr wahrscheinlich ist, so kommen grundsätzlich mindestens vier verschiedene Lösungsmöglichkeiten in Frage:
1. Der verstopfte Zentralkatheter wird aus dem Aussenkatheter herausgezogen, durch einen neuen Zentralkatheter ersetzt, der neue Zentralkatheter wird im Aussenkatheter vorgeschoben und der Absaugvorgang mit dem neuen Zentralkatheter fortgesetzt.
2. Der verstopfte Zentralkatheter wird aus dem Aussenkatheter herausgezogen, anstelle dessen der Aussenkatheter an die Absaugvorrichtung angeschlossen und der Absaugvorgang über den Aussenkatheter fortgesetzt.
3. Der verstopfte Zentralkatheter wird aus dem Aussenkatheter herausgezogen, die Verstopfung aus dem Zentralkatheter entfernt und der gleiche Zentralkatheter wieder eingeführt und der Absaugvorgang mit dem gleichen Zentralkatheter fortgesetzt.
4. Nach dem Verstopfen des Zentralkatheters wird der Aussenkatheter, unter Beibehaltung des Unterdrucks am Zentralkatheter, vorgeschoben und auf diese Weise das gesamte Blutgerinnsel in den Aussenkatheter hineingezogen und anschliessend durch gemeinsames Herausziehen der beiden Katheter das gesamte Blutgerinnsel entfernt.

Dieses unter den Punkten 1 bis 3 beschriebene Einführen und Herausziehen des Zentralkatheters kann durch das Vorhandensein eines Aussenkatheters mehrmals wiederholt werden, ohne dass dadurch die Blutgefässe zusätzlich strapaziert werden. Auch kann durch den Aussenkatheter der Zentralkatheter sehr schnell herausgezogen und wieder eingeführt werden. Durch den im Gefäss verbleibenden Aussenkatheter wird der Zentralkatheter sicher wieder an dieselbe Behandlungsstelle geführt. Es wird keine Zeit verbraucht um die Behandlungsstelle wieder neu aufzufinden. Der vorgeschlagene Katheter ist auch sehr einfach im Aufbau, er besteht lediglich aus zwei ineinander passenden Schläuchen mit einem innen liegenden Dilator. Die Teile müssen keinen hohen Belastungen standhalten. Der neue Katheter ist daher sehr einfach herzustellen.

Ein weiterer wichtiger Vorteil einer solchen Katheteranordnung ist, dass diese, im Vergleich zu Katheteranordnungen mit einem Rotationskörper, sehr kostengünstig herzustellen ist. Auch braucht keine Antriebsvorrichtung für den Rotationskörper vorhanden zu sein. Ebenso wird der innerhalb des Katheters vorhandene Querschnitt nicht unnötig eingeschränkt. Durch die kostengünstige Herstellung wird der Einmalgebrauch einer solchen Katheteranordnung zudem begünstigt und es entfällt daher eine aufwendige, zeitraubende und für die eingesetzten Materialien nicht immer ganz unproblematische Neusterilisierung der gebrauchten Katheteranordnung. Die zuverlässige Reinigung von Innenlumen von Kathetern ist im allgemeinen schwierig.

Um auch das Anschliessen des Aussenkatheters an eine Absaugvorrichtung zu ermöglichen, wird in einer weiteren, bevorzugten Ausführungsform vorgeschlagen, dass der Aussenkatheter im Bereich seines proximalen Endes über ein Anschlussteil verfügt, mittels welchem er an eine Absaugvorrichtung anschliessbar sind. Durch ein Anschliessen des Aussenkatheters an eine Absaugvorrichtung wird ermöglicht, dass nach dem Zurückziehen des Zentralkatheters aus dem Aussenkatheter, der Aussenkatheter selber mit Unterdruck beaufschlagt wird und somit über den Aussenkatheter das Blutgerinnsel bzw. zurückgebliebene Reste davon abgesaugt werden.

Eine weitere Ausführungsform der Katheteranordnung sieht mehrere Aussenkatheter vor. Dadurch ist es möglich, dass nach dem Zurückziehen des Zentralkatheters einfach der Innerste der Aussenkatheter zum Absaugen eingesetzt wird. Durch eine solche Ausgestaltung können mehrere Katheter nacheinander zum Einsatz gebracht werden, ohne dass dafür jeweils ein neuer Zentralkatheter eingeführt werden muss. Andererseits können dadurch am proximalen Ende der Kathetervorrichtung hohe Steifigkeiten erreicht werden, ohne dass die Flexibilität, der grosse Absaugquerschnitt und der kleine Aussendurchmesser des distalen Endes beeinträchtigt wird.

Bei einer weiteren, bevorzugten Ausgestaltung der Erfindung ist vorgesehen, dass der Aussendurchmesser des Zentralkatheters zumindest annähernd dem Innendurchmesser des Aussenkatheters entspricht, bzw. bei mehreren Aussenkathetern der Aussendurchmesser des inneren Aussenkatheters jeweils dem Innendurchmesser des nächst äusseren Aussenkatheters entspricht. Dadurch wird erreicht, dass der Aussendurchmesser des Aussenkatheters nur unwesentlich grösser als der des Zentralkatheters ist, und dass die Katheteranordnung dadurch nicht unnötig im Durchmesser vergrössert wird. Ausserdem wird durch eine solche Ausgestaltung der Zentralkatheter im Aussenkatheter geführt. Es versteht sich von selbst, dass die Durchmesser dabei so gewählt werden, dass die Katheter dennoch leicht axial gegeneinander verschiebbar sind. Auch kann durch das enge Umschliessen, die Wandstärke der beiden bzw. der verschiedenen Katheter unterschiedlich ausgebildet sein, und zwar kann diese so dünn gewählt werden, dass die Wandstärke des einen Katheters alleine keine genügend hohe Steifigkeit zum sicheren Einführen in ein Blutgefäss aufweisen würde, dass die ineinander geschobenen Katheter jedoch zusammen wieder eine genügende Steifigkeit bzw. Knickfestigkeit besitzen.

Um diese Verschiebbarkeit zu unterstützen, wird in einer weiteren Ausführungsform als Material für die Katheter Teflon vorgeschlagen.

Eine weitere Ausführungsform sieht vor, dass die Spitze des Dilators mit der Achse des Schaftes eine Winkel einschliesst, und dass die Spitze des Dilators durch Drehung des Dilators an dessen proximalem Ende in verschiedene Richtungen drehbar ist. Dadurch ist der Dilator steuerbar, so dass auf diese Weise selbst verschlungene Gefässwindungen sicher erreicht werden können.

Schliesslich ist in einer weiteren Ausgestaltung noch vorgesehen, dass die Spitze des Dilators gegenüber der Achse des Schaftes gebogen werden kann. Auf diese Weise kann die Spitze individuell steuerbar oder gerade ausgebildet werden.

Im folgenden wird ein Ausführungsbeispiei der Erfindung anhand der beiliegenden Zeichnung näher erläutert. In der Zeichnung zeigt:
Fig. 1 eine perspektivische Ansicht der Aspirationskatheteranordnung mit eingelegtem Dilator und Führungdraht;
Fig. 2 eine perspektivische Ansicht der Aspirationskatheteranordnung in einer Ausführung mit zwei Aussenkathetern; und
Fig. 3 eine perspektivische, schematische Ansicht der Aspirationskatheteranordnung beim Absaugen eines Blutgerinnsels.

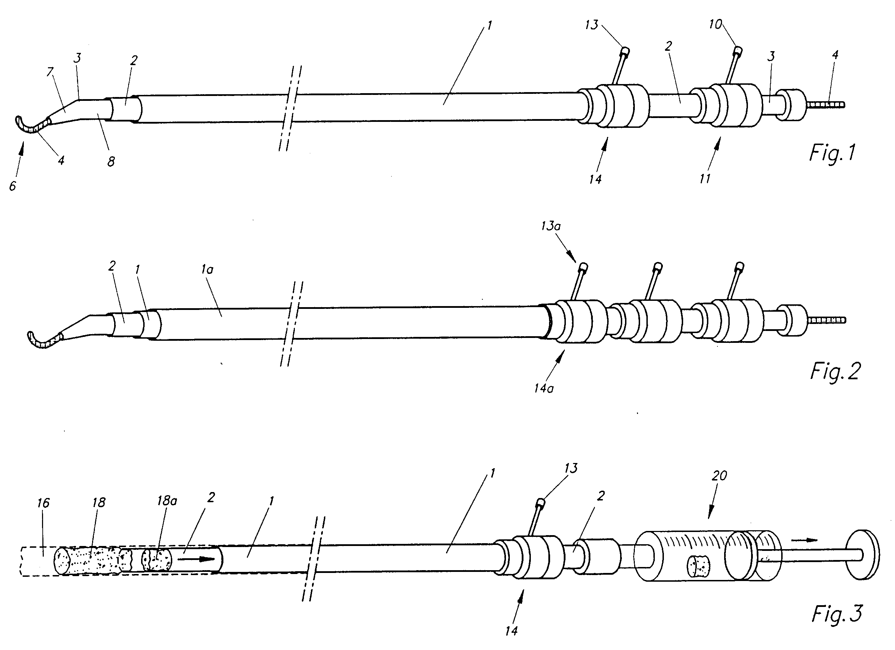

Fig. 1 zeigt in einer perspektivischen Ansicht die Aspirationskatheteranordnung. Die wesentlichen Bestandteile dieser Katheteranordnung sind ein Aussenkatheter 1, ein Zentralkatheter 2, ein Dilator 3 und ein Führungsdraht 4. Der Führungsdraht 4 ist in den Dilator 3 eingeschoben. Führungsdrähte 4 sind an ihrem distalen Ende so flexibel gehalten, dass sie mit Sicherheit beim Vorschieben im Gefäss nicht die Gefässwand verletzen. Führungsdrähte 4 sind ausserdem aber auch möglichst torsionssteif und ihr distales Ende lässt sich bleibend verbiegen. Mit Hilfe dieser Eigenschaften lässt sich der Führungdraht 4 von seinem proximalen Ende her beispielsweise in eine Abzweigung im Gefässsystem dirigieren.

Der Dilator 3 seinerseits ist rohrförmig ausgebildet und weist an seinem distalen Ende eine konisch ausgebildete Spitze 7 auf, deren Achse mit der Achse des Schaftes 8 einen Winkel einschliesst. Diese Spitze 7 kann gegenüber der Achse des Schaftes 8 gebogen und dadurch individuell der Aufgabenstellung angepasst werden. Durch drehen des Dilators 3 an dessen proximalem Ende kann die Spitze 7 in verschiedene Richtungen gedreht werden. Der Zentralkatheter 2 umschliesst den Dilator 3 auf einem Grossteil von dessen Längsausdehnung und weist an seinem proximalen Ende ein Anschlussteil 10 auf, beispielsweise zum Eingeben von Kontrastflüssigkeit mit einem hämostatischen Ventil 11. Der Zentralkatheter 2 seinerseits wird auch auf einem Grossteil seiner Längsausdehnung vom Aussenkatheter 1 umschlossen, wobei der Aussenkatheter 1 an seinem distalen Ende ebenfalls über ein Anschlussteil 13 und über ein hämostatisches Ventil 14 verfügt.

Fig. 2 zeigt eine weitere Ausführungsform der Aspirationskatheteranordnung, wobei der Unterschied zur Ausführung nach Fig. 1 darin besteht, dass ein zweiter Aussenkatheter 1a vorhanden ist, welcher den ersten Aussenkatheter 1 auf einem Grossteil seiner Längsausdehnung umfasst. Dieser Aussenkatheter 1a verfügt an seinem proximalen Ende ebenfalls über ein Anschlussteil 13a und über ein hämostatisches Ventil 14a. Natürlich sind auch Ausführungen möglich, die noch weitere Aussenkatheter aufweisen.

Das Ausbringen eines Blutgerinnsels wird anhand der Fig. 3 erläutert. Bei der in dieser Figur dargestellten Katheteranordnung ist deren Zentralkatheter 2 im vordersten, distalen Bereich, zur besseren Veranschaulichung im Längsschnitt dargestellt. Die Einführung einer solchen Katheteranordnung in ein Blutgefäss ist bekannt, weshalb hier nur auf die wichtigsten Punkte eingegangen wird. Durch eine Punktion in der Haut wird als erstes der Führungsdraht 4 in ein Blutgefäss 16 gelegt und beispielsweise bis in die Nähe des zu entfernenden Blutgerinnsels 18, 18a vorgeschoben. Danach wird der Dilator 3 zusammen mit dem Zentralkatheter 2 und dem Aussenkatheter 1 in das Blutgefäss 16 eingeführt und soweit wie nötig vorgeschoben, wobei der Dilator 3 den Zentralkatheter 2, zum Aufweiten der Punktionsstelle distal etwas überragt. Die an den proximalen Enden der Katheter 1, 2 angebrachten, hämostatischen Ventile 11, 14, verhindern dabei ein Ausfliessen von Blut über die Katheteranordnung.

Das Einführen und Vorschieben der beiden Katheter 1, 2 kann auf unterschiedliche Weise erfolgen. So können bei Katheter 1, 2 und der Dilator 3 zusammen und mit distal fluchtenden Enden eingeführt werden. Es ist aber auch möglich, dass beim Einführen das distale Ende des einen Katheters, vorzugsweise des Zentralkatheters 2, vor dem distalen Ende des anderen Katheters liegt. Dadurch kann die Steifigkeit und der Durchmesser der Katheteranordnung im Bereich ihres distalen Endes variiert werden.

Schliesslich wird der Zentralkatheter 2 ganz an das zu entfernende Blutgerinnsel 18 herangeführt. Spätestens jetzt wird der Dilator 3 zusammen mit dem Führungsdraht 4 aus dem Zentralkatheter 2 herausgezogen und entfernt. Das Entfernen des Blutgerinnsels 18, 18a geschieht auf herkömmliche Weise durch Absaugen. Dazu wird das hämostatische Ventil 11 des Zentralkatheters 2 entfernt und der Zentralkatheter 2 am Anschlussteil 10 an eine manuelle Absaugvorrichtung 20 angeschlossen. Anstelle einer manuellen Absaugvorrichtung 20, kommen natürlich auch andere Lösungen, wie beispielsweise eine elektrisch angetriebene Pumpe, in Frage. Durch den im Zentralkatheter 2 herrschenden Unterdruck, kann nun das Blutgerinnsel 18, 18a kontinuierlich abgesaugt werden. Sollte der Zentralkatheter 2 beim Absaugen, durch das Blutgerinnsel 18 oder einen Teil 18a davon, verstopfen, so kann der Zentralkatheter 2 aus dem Aussenkatheter 1 herausgezogen und gereinigt werden, oder es kann ein neuer Zentralkatheter in den Aussenkatheter 1 eingeschoben und nach dessen Anschliessen an die Absaugvorrichtung 20 damit weiter gearbeitet werden. Es ist jedoch auch möglich, den Absaugvorgang nach dem Entfernen des Zentralkatheters 2 mit dem Aussenkatheter 1 weiterzuführen, indem dessen hämostatisches Ventil 14 entfernt wird und das proximale Ende an die Absaugvorrichtung 20 angeschlossen wird.

Eine weitere Variante zum Ausbringen des Blutgerinnsels 18, 18a besteht darin, dass versucht wird, dieses durch einen am Zentralkatheter 2 anliegenden Unterdruck und eine Vorwärtsbewegung des Aussenkatheters 1 gesamthaft in den Aussenkatheter 1 hineinzuziehen und danach durch gleichzeitiges Herausziehen der beiden Katheter 1, 2, vorzugsweise unter Beibehaltung des Unterdrucks am Zentralkatheter 2, zu entfernen. Dies ist insofern unproblematisch, da das Blutgerinnsel 18, 18a nach dem Hineinziehen in den Aussenkatheter 2, beim Herausziehen der Katheteranordnung aus dem Blutgefäss 16, keiner Reibung mehr ausgesetzt ist und daher sicher entfernt werden kann. Diese Methode wird besonders wirksam, wenn zum Sichern des Thrombus am oder im Aussenkatheter 1 über das Anschlussteil 13 Unterdruck auch noch an den Aussenkatheter 1 angelegt wird.

Kommt eine Aspirationskatheteranordnung wie in der Fig. 2 dargestellt zum Einsatz, so sind noch weitere Varianten dieses Absaug-Verfahrens möglich. So kann nach dem Verstopfen des Zentralkatheters 2 und Entfernen desselben, der Absaugvorgang mit dem innersten Aussenkatheter 1 (Fig. 2) weitergeführt werden und nach allfälligem Verstopfen dieses Aussenkatheters 1 und Entfernen desselben, mit dem nächstäusseren Aussenkatheter 1a (Fig. 2) weitergearbeitet werden.

Im Gegensatz zu herkömmlichen Vorrichtungen zum Entfernen von Thromben und Emboli, weist eine solche Aspirationskatheteranordnung den grossen Vorteil auf, dass beim Verstopfen des Zentralkatheters 2, nach einem kurzen Unterbruch weitergearbeitet werden kann. Ein Verstopfen des Katheters brachte es bisher mit sich, dass dieser aus dem Blutgefäss entfernt werden musste Wurde das Blutgerinnsel bis zum Zeitpunkt des Verstopfens noch nicht vollständig entfernt, so musste ein neuer Katheter eingeführt werden. Bei einem erneuten Einführen kommen jedoch wieder die üblichen Probleme zum Tragen. So werden die Blutgefässe des Patienten ein weiteres Mal beansprucht und der Katheter muss wiederum vorsichtig an die richtige Stelle des Blutgefässes geführt werden. Dies ist inbesondere bei weit von der Punktionsstelle entfernt liegenden Blutgerinnseln sehr aufwendig. Auch geht in einem solchen Fall unnötig viel wertvolle Zeit verloren.

Abschliessend kann gesagt werden, dass mit der erfindungsgemässen Aspirationskatheteranordnung ein Blutgerinnsel in der kürzest möglichen Zeit entfernt werden kann, und dass dabei die Blutgefässe des Patienten geschont werden.

## Patentansprüche

1. Aspirationskatheteranordnung zum Entfernen von Thromben und Emboli aus Blutgefässen durch kontinuierliche Absaugung, mit einem Zentralkatheter (2), der zur Aufnahme eines axial verschiebbaren Dilators (3) ausgebildet ist sowie einem den Zentralkatheter (2) koaxial umfassenden Aussenkatheter (1), wobei die Aspirationskatheteranordnung zum kontinuierlichen Absaugen von Thromben und Emboli ausgebildet ist, indem der Zentralkatheter (2) während der kontinuierlichen Absaugung einen bis zu seinem distalen Ende gleichbleibenden Innendurchmesser aufweist, wobei das distale Ende des Aussenkatheters (1) bis an das distale Ende des Zentralkatheters (2) heranschiebbar ist, und wobei der Aussendurchmesser des Aussenkatheters (1) nur unwesentlich grösser als der des Zentralkatheters (2) ist, so dass der zum Absaugen zur Verfügung stehende Querschnitt annähernd dem Querschnitt des Aussenkatheters (1) entspricht.

2. Aspirationskatheteranordnung nach Anspruch 1, dadurch gekennzeichnet, dass der Aussenkatheter (1) im Bereich seines proximalen Endes ein Anschlussteil (13) aufweist, mittels welchem der Aussenkatheter (1) an eine Absaugvorrichtung (20) anschliessbar ist.

3. Aspirationskatheteranordnung nach Anspruch 1 oder 2, dadurch gekennzeichnet, dass der Aussenkatheter (1) und der Zentralkatheter (2) im Bereich ihrer proximalen Enden jeweils über ein hämostatisches Ventil (11, 14) verfügen.

4. Aspirationskatheteranordnung nach Anspruch 3, dadurch gekennzeichnet, dass das hämostatische Ventil (11, 14) lösbar am Anschlussteil (10, 13) befestigt ist.

5. Aspirationskatheteranordnung nach einem der vorhergehenden Ansprüche, dadurch gekennzeichnet, dass eine Mehrzahl von Aussenkathetern (1, 1a) vorgesehen ist, die koaxial zum Zentralkatheter (2) angeordnet sind.

6. Aspirationskatheteranordnung nach Anspruch 5, dadurch gekennzeichnet, dass jeweils der Aussendurchmesser des inneren Aussenkatheters (1) zumindest annähernd dem Innendurchmesser des nächst äusseren Aussenkatheters (1a) entspricht.

7. Aspirationskatheteranordnung nach einem der vorhergehenden Ansprüche, dadurch gekennzeichnet, dass die Katheter (1, 1a, 2) aus Teflon bestehen.

8. Aspirationskatheteranordnung nach einem der vorhergehenden Ansprüche, dadurch gekennzeichnet, dass ein Dilator (3) vorgesehen ist, der zur Aufnahme in einem Zentralkatheter (2) ausgebildet ist und eine Spitze (7) aufweist, welche mit ihrer Achse gegenüber der Achse des Schaftes (8) einen Winkel einschliesst, und dass die Spitze (7) des Dilators (3) durch Drehung des Dilators in verschiedene Richtungen drehbar ist.

9. Aspirationskatheteranordnung nach Anspruch 9, dadurch gekennzeichnet, dass die Spitze (7) des Dilators (3) mit ihrer Achse bleibend in einen Winkel gegenüber der Achse des Schaftes (8) biegbar ist, und dass die Spitze (7) durch Drehung des Dilators (3) in verschiedene Richtungen drehbar ist.

10. Aspirationskatheteranordnung nach Anspruch 8 oder 9, dadurch gekennzeichnet, dass im Dilator (3) ein Führungsdraht (4) geführt ist.

## Claims

1. Suction catheter arrangement for removing thrombi and emboli from blood vessels by continuous suctioning, having a central catheter (2) which is designed to receive an axially displaceable dilator (3) and an outer catheter (1) which coaxially surrounds the central catheter (2), the suction catheter arrangement being designed for continous suctioning of thrombi and emboli, and the central catheter (2) has, during the continuous suctioning, an internal diameter which remains constant up to its distal end, and the distal end of the outer catheter (1) can be pushed as far as the distal end of the central catheter (2), and the external diameter of the outer catheter (1) is only slightly greater than that of the central catheter (2), so that the cross-section available for the suctioning corresponds approximately to the cross-section of the outer catheter (1).

2. Suction catheter arrangement according to Claim 1, characterized in that the outer catheter (1) has, in the area of its proximal end, a connection part (13) by means of which the outer catheter (1) can be connected to a suction device (20).

3. Suction catheter arrangement according to Claim 1 or 2, characterized in that the outer catheter (1) and the central catheter (2) have, in the area of their proximal ends, in each case a haemostatic valve (11, 14).

4. Suction catheter arrangement according to Claim 3, characterized in that the haemostatic valve (11, 14) is fixed detachably to the connection part (10, 13).

5. Suction catheter arrangement according to one of the preceding claims, characterized in that a plurality of outer catheters (1, 1a) are provided and are arranged co-axially to the central catheter (2).

6. Suction catheter arrangement according to Claim 5, characterized in that in each case the external diameter of the inside outer catheter (1) corresponds at least approximately to the internal diameter of the outer catheter (1a) next outside.

7. Suction catheter arrangement according to one of the preceding claims, characterized in that the catheters (1, 1a, 2) consist of Teflon.

8. Suction catheter arrangement according to one of the preceding claims, characterized in that a dilator (3) is provided which is designed to be received in a central catheter (2) and has a tip (7) which, with its axis, forms an angle relative to the axis of the shaft (8), and in that the tip (7) of the dilator (3) can be turned by turning the dilator in different directions.

9. Suction catheter arrangement according to Claim 9, characterized in that the tip (7) of the dilator (3) can be bent, with its axis remaining at an angle relative to the axis of the shaft (8), and in that the tip (7) can be turned by turning the dilator (3) in different directions.

10. Suction catheter arrangement according to Claim 8 or 9, characterized in that a guide wire (4) is guided in the dilator (3).

## Revendications

1. Dispositif formant sonde d'aspiration pour retirer des thrombus et des emboles des vaisseaux sanguins par aspiration continue, comprenant une sonde centrale (2) réalisée de manière à recevoir un dilatateur (3) qui peut coulisser dans le sens axial ainsi qu'une sonde externe (1) dans laquelle la sonde centrale (2) est disposée coaxialement, caractérisé en ce que ledit dispositif formant sonde d'aspiration est réalisé pour aspirer en continu des thrombus et des emboles, la sonde centrale (2) présentant au cours de l'aspiration en continu un diamètre intérieur qui reste identique jusqu'à son extrémité distale, en ce que l'extrémité distale de la sonde externe (1) peut coulisser jusqu'à l'extrémité distale de la sonde centrale (2) et en ce que le diamètre extérieur de la sonde externe (1) est à peine supérieur à celui de la sonde centrale (2), de sorte que la section disponible pour l'aspiration correspond approximativement à la section de la sonde externe (1).

2. Dispositif formant sonde d'aspiration selon la revendication 1, caractérisé en ce que la sonde externe (1) présente dans la zone de son extrémité proximale une partie de raccordement (13) au moyen de laquelle la sonde externe (1) peut être raccordée à un dispositif d'aspiration (20).

3. Dispositif formant sonde d'aspiration selon la revendication 1 ou la revendication 2, caractérisé en ce que la sonde externe (1) et la sonde centrale (2) sont munies l'une et l'autre dans la zone de leurs extrémités proximales d'une valve hémostatique (11, 14).

4. Dispositif formant sonde d'aspiration selon la revendication 3, caractérisé en ce que la valve hémostatique (11, 14) est fixée de manière amovible à la partie de raccordement (10, 13).

5. Dispositif formant sonde d'aspiration selon l'une quelconque des revendications précédentes, caractérisé en ce qu'il est prévu une multitude de sondes externes (1, 1a) qui sont disposées coaxialement par rapport à la sonde centrale (2).

6. Dispositif formant sonde d'aspiration selon la revendication 5, caractérisé en ce que le diamètre extérieur de la sonde externe (1) intérieure correspond au moins approximativement au diamètre intérieur de la sonde interne (1a) immédiatement extérieure.

7. Dispositif formant sonde d'aspiration selon l'une quelconque des revendications précédentes, caractérisé en ce que les sondes (1, 1a, 2) sont en téflon.

8. Dispositif formant sonde d'aspiration selon l'une quelconque des revendications précédentes, caractérisé en ce qu'il est prévu un dilatateur (3) réalisé de manière à être reçu dans une sonde centrale (2) et présentant une pointe (7) dont l'axe fait un angle par rapport à l'axe de la tige (8) et en ce que l'on peut faire tourner la pointe (7) du dilatateur (3) dans différentes directions en faisant tourner le dilatateur.

9. Dispositif formant sonde d'aspiration selon la revendication 8, caractérisé en ce que la pointe (7) du dilatateur (3) peut être pliée avec son axe restant dans un certain angle par rapport à l'axe de la tige (8) et en ce que l'on peut faire tourner la pointe (7) du dilatateur (3) dans différentes directions en faisant tourner le dilatateur.

10. Dispositif formant sonde d'aspiration selon la revendication 8 ou la revendication 9, caractérisé en ce qu'un fil de guidage (4) est guidé dans le dilatateur (3).
